(19) Europäisches Patentamt
European Patent Office
Office européen des brevets

(11) Publication number : **0 687 673 A1**

(12) # EUROPEAN PATENT APPLICATION

(21) Application number : **95304021.9**

(51) Int. Cl.[6] : **C07D 263/32,** A61K 31/42

(22) Date of filing : **09.06.95**

(30) Priority : **14.06.94 GB 9411841**

(43) Date of publication of application : **20.12.95 Bulletin 95/51**

(84) Designated Contracting States : **BE CH DE DK ES FR GB IT LI NL SE**

(71) Applicant : **PHARMA MAR, S.A.**
**Calle de la Calera 3,**
**Poligono Industrial de Tres Cantos**
**E-28760 Tres Cantos, Madrid (ES)**

(72) Inventor : **Gravalos, Dolores G c/o Pharma Mar, S.A.**
**Calle de la Calera No. 3**
**Poligono Industriale**
**E-28760 Tres Cantos, Madrid (ES)**
Inventor : **Kashman, Yoel**
**School of Chemistry**
**Tel-Aviv University**
**Tel-Aviv 69978 (IL)**
Inventor : **Rudi, Amira**
**School of Chemistry**
**Tel-Aviv University**
**Tel-Aviv 69978 (IL)**
Inventor : **De La Fuente, Jesus Angel c/o Pharma Mar S.A.**
**Calle de la Calera 3**
**Poligono Industriale**
**E-28760 Tres Cantos, Madrid (ES)**

(74) Representative : **Ruffles, Graham Keith**
**MARKS & CLERK,**
**57-60 Lincoln's Inn Fields**
**London WC2A 3LS (GB)**

(54) **Oxazole derivatives as antitumoral agents**

(57) New antitumoral compounds are of formula (I) :

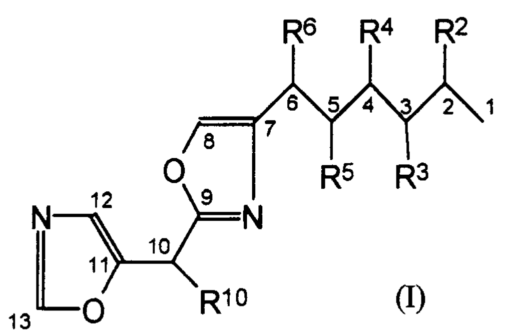

(I)

where $R^2$, $R^3$, $R^4$, $R^5$, $R^6$, and $R^{10}$ are the same or different and each represents a hydrogen atom, an alkyl group of 1 to 6 carbon atoms, a hydroxy group or an acyloxy group R-(C=O)-O- (where R-(C=O)- is an acyl group of 1 to 26 carbon atoms) ; provided that at least one of $R^2$, $R^3$, $R^4$, $R^5$, $R^6$, and $R^{10}$ is an acyloxy group R-(C=O)-O- (where R-(C=O)- is an acyl group of 1 to 26 carbon atoms) and further provided that at least one of $R^2$, $R^3$, $R^4$, $R^5$, $R^6$, and $R^{10}$ is an acyloxy group R-(C=O)-O- (where R-(C=O)- is an acyl group of 10 to 25 carbon atoms) ; with the exception of the tetraacetate of bengazole A and the

EP 0 687 673 A1

Jouve, 18, rue Saint-Denis, 75001 PARIS

tetraacetate of bengazole B. The lead to these compounds is provided by digonazole triacetate of formula (V), obtained from digonazole which may itself be extracted from *Jaspis digonoxea*.

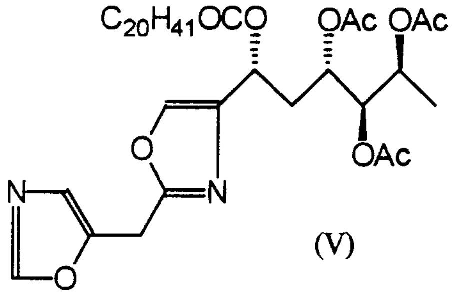

(V)

This invention is concerned with new antitumoral compounds. In particular, the invention is concerned with antitumor compounds which may be obtained by derivatisation of a compound isolated from a *Jaspis* sponge, and related antitumor compounds.

## Background of the Invention

Marine organisms provide a potential source of biologically active molecules, including compounds of potential use as antitumor agents. Crews, P. *et al.* in *J. Am. Chem. Soc.* 1988, 110, 1598-1602 [Reference 1] and in *J. Nat. Prod.* **1993**, 56, 2034-2040 [Reference 2], report on new structures and bioactivity patterns of bengazole alkaloids from sponges of the Jaspidae family. Some bengazoles were evaluated for cytotoxicity. The parent compound, bengazole A, was known to have shown *in vitro* potency against two human tumour cell lines.

## Object of the Invention

It is an object of this invention to provide new compounds of potential use as antitumor agents. A related object is the isolation of compounds of interest from marine organisms and also the synthesis of such compounds.

## Summary of the Invention

We have isolated from the known Indo-Pacific sponge *Jaspis digonoxea* (De Laubenfels, 1950; Class Demospongiae, Order Choristida, family Jaspidae) a new marine natural product designated digonazole. This compound has antitumor activity, which can be increased by derivatisation and which thus indicates a lead to further active antitumour agents.

Accordingly the invention provides compounds of the formula (I):

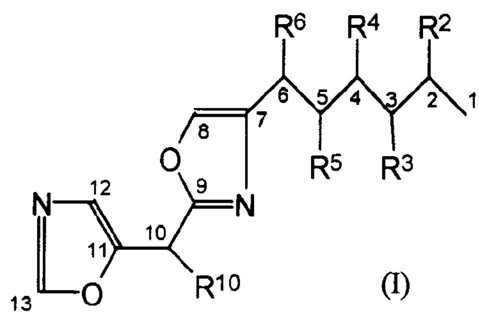

(I)

where $R^2$, $R^3$, $R^4$, $R^5$, $R^6$, and $R^{10}$ are the same or different and each represents a hydrogen atom, an alkyl group of 1 to 6 carbon atoms, a hydroxy group or an acyloxy group R-(C=O)-O- (where R-(C=O)- is an acyl group of 1 to 25 carbon atoms); provided that at least one of $R^2$, $R^3$, $R^4$, $R^5$, $R^6$, and $R^{10}$ is an acyloxy group R-(C=O)-O- (where R-(C=O)- is an acyl group of 1 to 26 carbon atoms) and further provided that at least one of $R^2$, $R^3$, $R^4$, $R^5$, $R^6$, and $R^{10}$ is an acyloxy group R-(C=O)-O- (where R-C=O)- is an acyl group of 10 to 26 carbon atoms); with the exception of the tetraacetate of bengazole A and the tetraacetate of bengazole B.

The invention also provides a pharmaceutical composition containing a compound of formula (I) in association with a pharmaceutical carrier or diluent. Further the invention provides the antitumoral use of a compound of Formula (I). Processes for preparing the compounds of formula (I) are provided.

## Preferred Embodiments of the Invention

The compounds of the present invention have groups $R^2$, $R^3$, $R^4$, $R^5$, $R^6$, and $R^{10}$ which are numbered by respective the carbon atom on which each is a substituent. At least two of the substituents $R^2$, $R^3$, $R^4$, $R^5$, $R^6$, and $R^{10}$ are acyloxy groups. One acyloxy group includes an acyl group of 1 to 26 carbon atoms, and may have either a short acyl group of 1 to 9 carbon atoms, or a long acyl group of 10 to 26 carbon atoms. A second acyloxy group includes a long acyl group of 10 to 26 carbon atoms. There may be further acyloxy groups with short or long acyl groups.

Examples of the alkyl group for $R^2$, $R^3$ $R^4$, $R^5$, $R^6$ and $R^{10}$ include methyl, ethyl, propyl, isopropyl and butyl groups. Examples of the acyloxy group R-(C=O)-O- for $R^2$, $R^3$, $R^4$, $R^5$, $R^6$, and $R^{10}$ include acetoxy, propoxy and other short acyloxy groups with 1 to 9 carbon atoms, more preferably 2 to 4 carbon atoms, as well as fatty

acyloxy groups with 10 to 26 carbon atoms, preferably straight chain groups. The acyloxy groups may be unsaturated alkenoyloxy groups, preferably with a single unsaturation in a straight chain, such as occur in natural fatty acids.

A particularly preferred group of compounds of this invention are of the formula (I), in which $R^2$, $R^3$ and $R^4$ are acetoxy or other short acyloxy groups, $R^5$ and $R^{10}$ are hydrogen atoms, and $R^6$ is a henicosanoyloxy group (that is, a group $C_{20}H_{41}$ COO-) or another fatty acyloxy group.

The compounds of this invention have optical centres, and the present invention extends to the individual isomers as well as mixtures including the racemic compounds.

Examples of pharmaceutical compositions provided by this invention include solid (tablets, pills, capsules, granules, etc.) or liquid (solutions, suspensions or emulsions) formulations with a suitable composition for oral, topical or parenteral administration. They may contain the pure compound or in combination with any other pharmacologically active compound. These compositions may need to be sterile when administered parentally.

The correct dosage of a pharmaceutical composition comprising a compound of formula (I) will vary according to the pharmaceutical formulation, the mode of application, and the particular situs, host and tumor being treated. Other factors like age, body weight, sex, diet, time of administration, rate of excretion, condition of the host, drug combinations, reaction sensitivities and severity of the disease shall be taken into account. Administration can be carried out continuously or periodically within the maximum tolerated dose.

Compounds of formula (I) can be made by synthetic procedures. An example of a suitable route is as follows, involving intermediates of formulae (II) and (III):

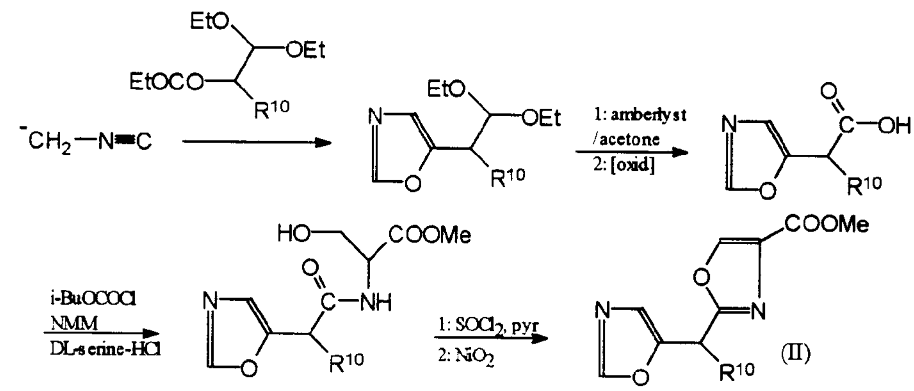


(II)

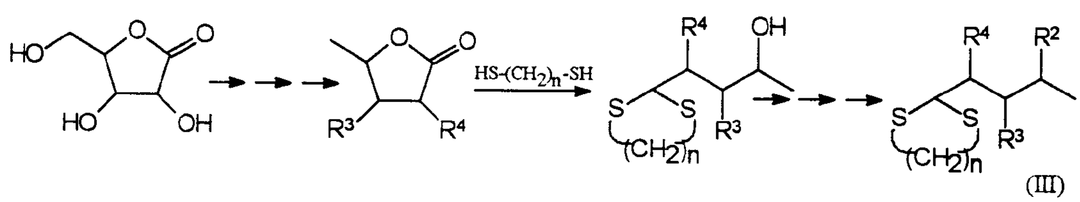


(III)

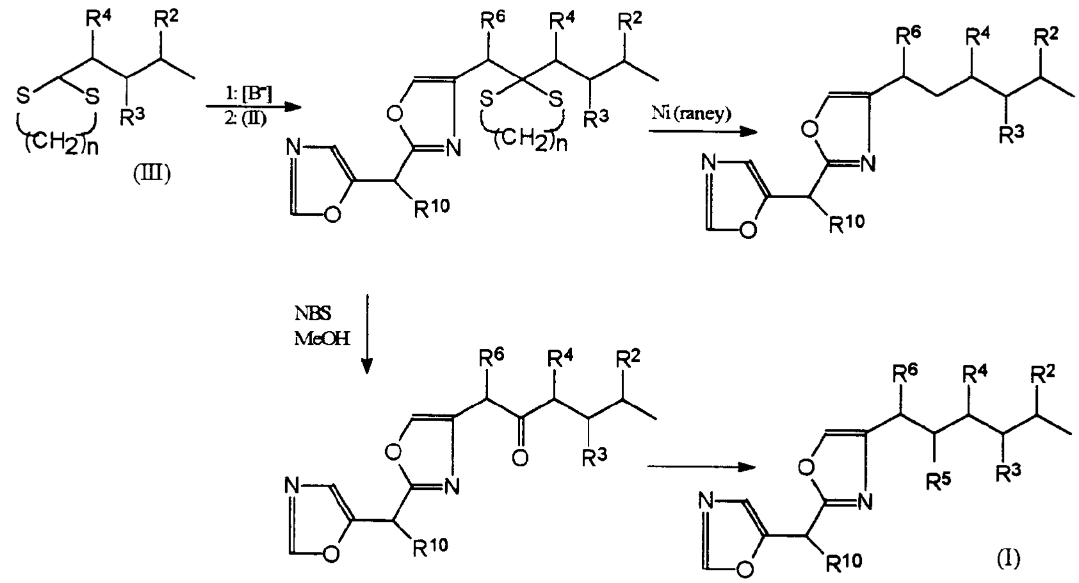


(III) (I)

A starting material of formula (IV) which we designate digonazole can be isolated from the sponge *Jaspis digonoxea*. Digonazole can reacted as desired to produce compounds of the present invention, notably digonazole triacetate of formula (V), as shown by the following reaction scheme:

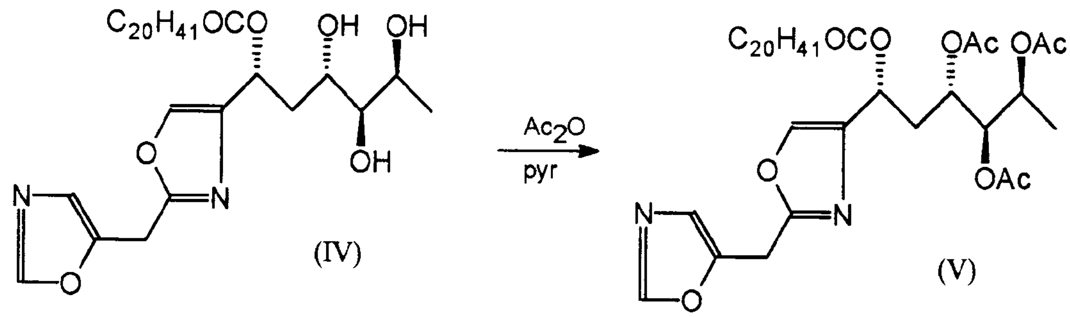


(IV) (V)

Diganozole can also be hydrolysed to give a tetrahydroxy compound of utility in preparing the compounds of this invention.

The compounds of this invention can be obtained as individual compounds free from other compounds of formula (I). In one embodiment, this invention provides the compounds in substantially pure form.

Examples of the Invention

IR spectra were recorded on a Nicolet 205 FT-IR spectrophotometer. Low resolution mass spectra were recorded on a Finnigan-4021 mass spectrometer. Hrms were taken on a VG70 VSEQ instrument. $^1H$ and $^{13}C$ nmr spectra were recorded on Bruker AMX-360 and ARX-500 spectrometers. All chemical shifts are reported with respect to TMS ($\delta$ = 0). Optical rotations were measured on a Perkin-Elmer Model 141 polarimeter using a 1 cm microcell.

*Jaspis digonoxea* (family Jaspidae) sponge was collected in Sodwana Bay, South Africa by divers using SCUBA (July 1992). A voucher specimen (TASA 110) is deposited in the zoological department at Tel-Aviv University. The freshly collected sponge was immediately frozen at -25°C. The freeze dried sponge (32g) was then extracted with ethyl acetate to give a brown gum (495 mg). The gum was chromatographed first over Sephadex LH-20 column eluted with $MeOH:CHCl_3$:hexane/1:1:2 and then several times over silica gel columns eluted with hexane-ethyl acetate mixtures (9:1: to 1:20) to afford **(1)** bengamide A (5 mg), **(2)** bengamide B (31 mg), **(4)** cyclo-(**L**-trans-[4-hydroxy-prolinyl]-L-phenylalanine) (5 mg), **(5)** a functionalized nonene lactone (5 mg), **(6)** digonazole (32 mg), and **(7)** cyclo-(L-prolinyl-L-tyrosine) (5 mg). Rf values (silica gel. ethyl acetate) **(1)** 0.3, **(2)** 0.32, **(4)** 0.20, **(5)** 0.95, **(6)** 0.25, **(7)** 0.22. Compounds **(1)**, **(2)**, **(4)** and **(5)** had the same spectral data as reported previously, see J. Am. Chem. Soc. 111, 647, (1989) [Reference 3] and J. Org. Chem. 55, 240,

(1990) [Reference 4].

Digonazole **(6)**, - Viscous oil; $[\alpha]^{20}_D$ 8.9° (c=2.2, $CHCl_3$); ir (neat) 3600, 1750, 1500 $cm^{-1}$; $^1H$ nmr ($CDCl_3$)δ 7.85 (s, IH, H-13), 7.65 (s, IH, H-8), 7.00(s, IH,H-12), 6.02 (dd, IH, J=9.6, 5.1 Hz, H-6), 4.20 (s, 2H, H-10), 4.03 (dq, IH,J=3.2,6.6 Hz, H-2), 3.80 (m, IH, H-4), 3.30 (t, 1H, J=3.0 Hz, H-3), 2.32 (t, 2H, J=7.0 Hz, H-15), 2.30 (ddd, IH, J=15.0, 9.6, 2.1, Hz, H-5), 2.10 (ddd, IH, J=15.0, 9.7, 5.1 Hz, H-5'), 1.60 (m, 2H, H-16), 1.25 (m, 32H), 1.24 (d, 3H, J=6.6 Hz, Me-1), 0.90 (t, 3H, J=6.6 Hz, Me-34), 0.88 (m, 2H); $^1H$ nmr (d4-MeOH) δ 8.00 (s, IH, H-13), 7.77 (s, IH, H-8), 6.98 (s, IH, H-12), 6.02 (dd, IH, J=9.6, 5.1 Hz, H-6), 4.21 (s, 2H, H-10),3.82 (dq, IH, J=3.1, 6.5 Hz, H-2), 3.36 (ddd, IH, J=2.1, 6.7, 10.4 Hz, H-4), 3.05 (dd, IH, J=3.3, 6.7 Hz, H-3), 2.35 (ddd, IH, J=15.4, 9.6, 2.1 Hz, H-5), 1.87 (ddd, IH, J=15.4, 10.1, 5.1 Hz, H-5'), 1.08(d, 3H, J=6.6 Hz, Me-34); $^{13}C$ nmr ($CDCl_3$) δ 173.1 (s, C-14), 159.5 (s, c-9), 151.1 (d, C-13), 145.6 (s, C-11), 138.9 (s, C-7), 137.7 (d, C-8), 124.4 (d, C-12), 77.0 (d, C-3), 69.9 (d, C-4), 67.0 (d, C-2), 65.7 (d, C-6), 36.2 (t, C-15), 31.9t, 29.7 (t, 10 CH), 29.4t. 29.2t, 29.1t, 29.0t, 25.2 (t-C-10), 24.8 (t, C-16), 22.7t, 19.5t, 14.0 (q, C-34).

HMBC Correlations, C to H: C-2/H-1, H-5; C-4/H-5', H-6; C-5/H-6; C-6/H-5; C-7/H-5, H-8; C-8/H-6; C-9/H-8, H-10; C-11/H-10; C-11/H-10, H-13; C-12/H-10, H-13; C-13/H-12; C-14/H-6, H-15, H-15', H-16, H-16'. hrcims ($MH^+$ 607.4332 ($C_{34}H_{59}N_2O_7$) (calc. 607.43225).

Triacetate Derivative of **(6)**, Compound **(8)**. - A solution of **(6)** (8 mg) in dry pyridine (0.5 ml) and acetic anhydride (0.5 ml) at room temperature was placed in the dark for 24 hr. The reaction mixture was concentrated in vacuo and the residue was chromatographed on silica gel (EtOAc: Hexane/1:1) to afford triacetate **(8)** in 90% yield; $[\alpha]_D^{20}$-8.0° (c=0.9, $CHCl_3$); $^1H$-nmr ($CDCl_3$) δ 7.96 (s, IH), 7.68 (s, IH), 7.13 (s, IH), 5.85 (dd, IH, J=6.0,9.6 Hz), 5.12 (m, 2H), 4.95 (dq, IH, J=3.1, 6.6 Hz), 4.30 (s, 2H), 2.24 (s, 3H), 2.16 (s, 3H), 2.11 (s, 3H), 1.25 (d, 3H, J=6.6 Hz), 0.99 (t 3H, J=6.6 Hz). The following H to H correlations have been observed in a COSY-45 spectrum (H/H): Me-1/H-2; H-3/H-4; H-4/H-5, H-5'; H-6/H-5, H-5'.

Tetrahydroxy Derivative of **(6)**, Compound **(9)**. A solution of **(6)** (10 mg) in 1% MeOH-KOH (1 ml) was placed in the dark for 24 hr. After being neutralized with a 1% solution of HCl, the mixture was partitioned between $H_2O$ (5 ml) and $CH_2Cl_2$ (3 x 5 ml). The combined organic extracts were dried over anhydrous $Na_2SO_4$ and concentrated to dryness to obtain the n-$C_{21}$-carboxylic acid (identified by characteristic nmr data and the eims of its methyl ester, m/z 340 ($M^+$)). The aqueous extract was concentrated in vacuo and the residue extracted with a 50% mixture of MeOH and $CH_3CN$ to yield after filtration and concentration an 80% yield of pure hydrolyzed product **(9)**; $[\alpha]_D^{20}$. 1°(c=0.1, MeOH); an oil; $^1H$-nmr (d4-MeOH: $CD_3CN$, 1:1)δ; 8.15 (s, IH), 7.74 (s, 1H), 7.09 (s, IH), 4.86 (m, IH), 4.28 (s, 2H), 3.99 (dd, IH, J=5.0,2.2 Hz), 3.72 (m, IH), 3.12 (m, 1H), 2,12 (ddd, IH, J=15.0, 9.7, 2.3 Hz), 1.88 (ddd, IH, J=15.0, 10.2, 5.1 Hz), 1.15 (d, 3H, J=6.6 Hz), (eims was performed on the tetraacetate derivative **(10)**.

Tetraacetate derivative of **(6)**, Compound **(10)**.- In the same procedure as described for **(6)**, compound **(9)** (4 mg), was acetylated to afford tetraacetate **(10)** in 90% yield; an oil; $^1H$-nmr ($CDCl_3$)δ: 7.75 (s, IH), 7.54 (s, IH), 6.92 (s, IH), 5.66 (dd, IH, J=5.3, 9.3 Hz), 5.01 (m, 2H), 4.77 (ddd, IH, J=9.9,4.3,2.3 Hz), 4.12 (s, 2H), 2.30 (ddd, IH, J=14.3, 9.2 2.4 Hz), 2.15 (ddd, IH, J=14.3, 9.8, 5.0 Hz), 2.04 (s, 3H), 1.96 (s, 3H), 1.93 (s, 3H), 1.91 (s, 3H), 1.06 (d, 3H, J=6.6 Hz); Ireims 466 ($M^+$, $C_{21}H_{26}O_{10}N_2$, 7%), 406 ($M^+$-60, 11%), 346, (17), 304 (23), 243 (50).

Digonazole had the composition $C_{34}H_{58}N_2O_7$ as deduced from the hrms and $^{13}C$-nmr. The $^1H$-nmr and $^{13}C$-nmr were similar to those of bengazole A, see J. Nat. Prod. 50, 994 (1987) [Reference 5] and J. Am Chem Soc. 110, 1598 (1988) [Reference 6]. Thus, the nmr data of **(6)** pointed clearly to two oxazole rings (Table 1) with the same substitution pattern as in compound **(3)** (see below). Most characteristics for the latter oxazoles, as already pointed out in Reference 6, were the various one- to three-bond carbon-hydrogen coupling constants (Table 1).

Table 1. Comparison of the nmr data of **(3)** and **(6)** in $CDCl_3$

| | **(6)** | | | **(3)** Ref 6 | | |
|---|---|---|---|---|---|---|
| atom number | $\delta_C$* | $\delta_H$ | $J_{CH}$ in Hz (with H#) | $\delta a_C$ | $\delta_H$ | $J_{CH}$ in Hz (with H#) |
| 7 | 138.9 | - | 10.8 (H-8) | 144.2s | - | 15.8 (H-8) |
| 8 | 137.7d (J=209) | 7.65s | - | 136.3d (211.7) | 7.66s | - |
| 9 | 159.5s | - | 8.0 (H-8), 8.0 (H-10) | 158.0s | - | 7.2 (H-8) |
| 11 | 145.6s | | multiplet | 147.6 | - | 18.4 (H-12) 4.4 (H-13) |
| 12 | 124.4d (195) | 7.00s | 5.8 (H-13) | 127.0d (197.8) | 7.21s | 7.3 (H-13) |
| 13 | 151.1d (222) | 7.85s | 8.2 (H-12) | 152.3d (233.6) | 7.94s | 10.5 (H-12) |

*: J in Hz.

From the HMBC experiment it was clear that the two oxazoles of **(6)** are connected to each other through a methylene ($\delta_c$25.2t, $\delta_H$4.2s(2H-10)) rather than a methinoxy group as in **(3)**. The latter methylene bridge was established by CH-correlations between $CH_2$-10 and C9, 11 and 12. Furthermore, home and hetero COSY correlations confirmed unequivocally a 2,3,4,6 - tetra oxygenated hexane chain ($\delta_H$ 6.02 (H-6), 4.03 (H-2), 3.80 (H-4), 3.30 (H-3), 2.30 (H-5), 2.10(H-5'), 1.24 (me-1)).

The lower-field resonance of H-6, in **(6)**, in comparison with the corresponding proton in **(3)**, suggested the C-6 methinoxy, (rather than the C-10 in **(3)**) to be esterified by a long-chain carboxylic acid. The location of the ester group, at C-6, was fully confirmed by CH-correlations between H6 ($\delta_H$6.02) and the ester CO group ($\delta_c$ 173.1). A $C_{21}$-fatty acid chain was evident from the m/z 325 (100%, $C_{21}H_{41}O_2^+$) fragment in the mass spectrum. The functionalization mode of the six carbon chain was further confirmed by a homo COSY experiment which was run on the 2,3,4-triacetate derivative of **(6)**, i.e. compound **(8)**. In the proton nmr spectrum of triacetate **(8)** the doublet of Me-1 moved up-field from δ 1.25 in 6, to δ 1.10, as a result its correlation with H-2 was observed clearly.

Mild basic hydrolysis of digonazole (1% KOH in MeOH, at room temperature) afforded the $C_{21}$-carboxylic acid and the tetrahydroxylhexyl dioxazole (formula (I), $R^2 = R^3 = R^4 = R^6$ = OH, $R^5 = R^{10}$ = H). For comparison purposes this compound was acetylated to the corresponding tetraacetate, **(10)**.

Biological Activity - Antitumor Assays

Cells were maintained in logarithmic phase of growth in Eagle's Minimum Essential Medium, with Earle's Balanced Salts, with 2.0 mM L-Glutamine, with non-essential Amino Acids, without Sodium Bicarbonate (EMEM/NEAA); supplemented with 10% Fetal Calf Serum (FCS), $10^{-2}$ M Sodium Bicarbonate and 0,1 g/1 Penicillin-G + Streptomycin Sulfate.

A simple screening procedure was carried out to determine and compare the antitumor activity of these compounds, using an adapted form of the method described by Bergeron *et al.*, Biochem. Biophys. Res. Comm. (1984) 121 848 [Reference 6]. The antitumor cell employed was P-388 (suspension culture of a lymphoid neoplasm from DBA/2 mouse), A-549 (monolayer culture of a human lung carcinoma), HT-29 (monolayer culture of a human colon carcinoma) and MEL-28 (monolayer culture of a human melanoma).

P-388 cell were seeded into 16 mm wells at 1 x $10^4$ cells per well in 1 ml aliquots of MEM 5FCS containing the indicated concentration of drug. A separate set of cultures without drug was seeded as control growth to ensure that cells remained in exponential phase of growth. All determinations were carried out in duplicate. After three days of incubation at 37°C, 10% $CO_2$ in a 98% humid atmosphere, an approximate $IC_{50}$ was determined by comparing the growth in wells with drug to the growth in control wells.

A-549, HT-29 and MEL-28 were seeded into 16 mm wells at 2 x $10^4$ cells per well in 1 aliquots of MEM 10FCS containing the indicated concentration of drug. A separate set of cultures without drug was seeded as control growth to ensure that cells remained in exponential phase of growth. All determinations were carried out in duplicate. After three days of incubation at 37°C, 10% $CO_2$ in a 98% humid atmosphere, the wells were stained with 0.1% Crystal Violet. An approximate $IC_{50}$ was determined by comparing the growth in wells with drug to the growth in control wells.

| Compound | | $IC_{50}$ (μg/ml) | | |
|---|---|---|---|---|
| | P388 | A-549 | HT-29 | MEL-28 |
| Digonazole **(6)** | 2.5 | 0.2 | 0.2 | |
| Digonazole triacetate **(8)** | 0.2 | 0.01 | 0.02 | 0.01 |
| Tetrahydroxyhexyl dioxazole **(9)** | >20 | 5 | 5 | 5 |
| Tetraacetoxyhexyl dioxazole **(10)** | 10 | 1 | 0.5 | 10 |

## Claims

1. A compound of the formula (I):

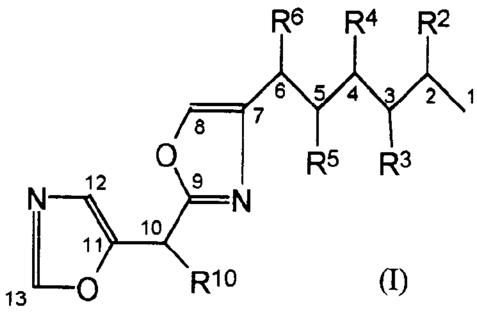

(I)

where $R^2$, $R^3$, $R^4$ $R^5$, $R^6$ and $R^{10}$ are the same or different and each represents a hydrogen atom, an alkyl group of 1 to 6 carbon atoms, a hydroxy group or an acyloxy group R-(C=O)-O- (where R-(C=O)- is an acyl group of 1 to 26 carbon atoms); provided that at least one of $R^2$, $R^3$, $R^4$, $R^5$, $R^6$ and $R^{10}$ is an acyloxy group R-(C=O)-O- (where R-(C=O)- is an acyl group of 1 to 26 carbon atoms) and further provided that at least one of $R^2$, $R^3$, $R^4$, $R^5$, $R^6$, and $R^{10}$ is an acyloxy group R-(C=O)-O- (where R-(C=O)- is an acyl group of 10 to 26 carbon atoms); with the exception of the tetraacetate of bengazole A and the tetraacetate of bengazole B.

2. A compound according to claim 1, which is of the formula (V):

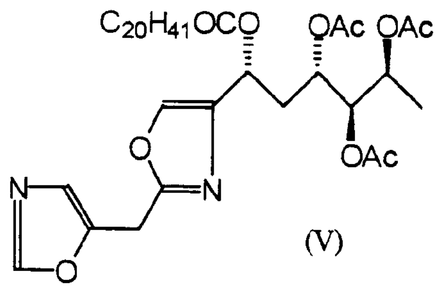

(V)

3. A compound according to claim 1, as an individual compound free from other compounds of formula (I).

4. A compound according to claim 1, in substantially pure form.

5. A compound according to claim 2, in substantially pure form.

6. A pharmaceutical composition containing a compound of formula (I), as defined in claim 1, in association with a pharmaceutical carrier or diluent.

7. A pharmaceutical composition containing a compound of formula (I), as defined in claim 3, in association with a pharmaceutical carrier or diluent.

8. A pharmaceutical composition containing a compound of formula (I), as defined in claim 4, in association with a pharmaceutical carrier or diluent.

9. A pharmaceutical composition containing a compound of formula (I), as defined in claim 5, in association with a pharmaceutical carrier or diluent.

10. Digonazole, a compound of formula (IV):

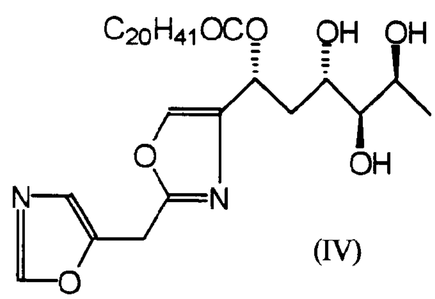

(IV)

European Patent Office

# EUROPEAN SEARCH REPORT

Application Number
EP 95 30 4021

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | JOURNAL OF NATURAL PRODUCTS, vol. 57, no. 6, June 1994 COLUMBUS,OHIO, pages 829-832, AMIRA RUDI ET AL 'Amino acid derivatives from the marine sponge Jaspis digonoxea' *page839,compounds 6 and 8,pages 831,832* | 1-5,10 | C07D263/32<br>A61K31/42 |
| A | US-A-4 785 012 (PHILIP CREWS ET AL) * the whole document * | 1,6 | |
| D,A | JOURNAL OF THE AMERICAN CHEMICAL SOCIETY, vol. 110, no. 5, 2 March 1988 DC US, pages 1598-1602, MADELINE ADAMCZESKI ET AL 'Unusual anthelminthic oxazoles from a marine sponge' * the whole document * | 1,6 | |
| D,A | JOURNAL OF NATURAL PRODUCTS, vol. 56 , no. 12, December 1993 COLUMBUS,OHIO, pages 2034-2040, JAIME RODRIGUEZ ET AL 'New structures and bioactivity patterns of bengazole alkaloids from a choristid marine sponge' * the whole document * | 1,6 | TECHNICAL FIELDS SEARCHED (Int.Cl.6)<br>C07D |

The present search report has been drawn up for all claims

| Place of search | Date of completion of the search | Examiner |
|---|---|---|
| THE HAGUE | 31 August 1995 | Henry, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)